# EUROPÄISCHE PATENTSCHRIFT

(11) **EP 0 753 502 B1**
(45) Veröffentlichungstag und Bekanntmachung des Hinweises auf die Patenterteilung: **18.08.1999**
(21) Anmeldenummer: 96107036.4
(22) Anmeldetag: 04.05.1996
(51) Int. Cl.: C07C 45/62, C07C 47/32

(54) **Verfahren zur Herstellung von 3.3.5-Trimethylcyclohexanon**
Process for the preparation of 3,3,5-trimethylcyclohexanone
Procédé de préparation de 3,3,5-triméthylcyclohexanone

(30) Priorität: 08.07.1995 DE 19524969
(43) Veröffentlichungstag der Anmeldung: 15.01.1997
(73) Patentinhaber: HÜLS AKTIENGESELLSCHAFT, 45764 Marl (DE)
(72) Erfinder: Büschken, Wilfried, Dr., 45721 Haltern (DE); Hummel, Jürgen, 45768 Marl (DE)

(56) Entgegenhaltungen:
- EP-A- 0 011 906
- US-A- 3 446 850

## Beschreibung

Die vorliegende Erfindung betrifft ein Verfahren zur Herstellung von 3.3.5-Trimethylcyclohexanon (TMC-on) durch katalytische Hydrierung von Isophoron.

TMC-on findet Verwendung für die Herstellung von Peroxiden, als Vorstufe für Riechstoffe, als Komponente von Lackharzen und als Lösemittel.

Ketone können aus ungesättigten Ketonen durch
a) Reduzierung mit komplexen Metallhydriden
b) Transfer-Hydrierung
c) Hydrierung mit Wasserstoff
hergestellt werden.

Die Methoden a) und b) haben den Nachteil, daß Koppelprodukte anfallen und mindestens ein Aufarbeitungsschritt notwendig ist.

Es ist bekannt, Isophoron an pulverförmigen Katalysatoren selektiv zu TMC-on zu hydrieren. So ist der JP 63 188 642 A zu entnehmen, daß hier Raney-Nickel als Katalysator und Isopropanol als Lösemittel verwendet wird. In der japanischen Patentschrift JP 47 016 434 wird als Katalysator Raney-Nickel und als Lösemittel Methanol offenbart. In den Patentschriften JP 51 024 497 und JP 7 602 494 werden Palladium-Katalysatoren mit verschiedenen Trägermaterialien beansprucht.

Ein Nachteil der obigen Verfahren ist, daß sie chargenweise durchgeführt werden. Nach jedem Ansatz muß der Katalysator abgetrennt werden, was neben dem Aufwand zu Handhabungsverlusten führt. Eine Wiederverwendung der Katalysatoren ist nur begrenzt möglich. Zudem ist für die Gewinnung des Hydrierproduktes bei Verwendung eines Lösemittels in der Hydrierstufe eine Destillation notwendig.

US 3 446 850 offenbart ein Verfahren zur Herstellung von 3,3,5-Trimethylcyclohexanon, wobei ein Feed gemisch aus 70 bis 90 Mol.-% 3,3,5-Trimethylcyclohexanon, 5 bis 15 Mol.-% 3,3,5-Trimethylcyclohexanol und 5 bis 15 Mol.-% Isophoron in Gegenwart eines Katalysators unter Druck bei einer Temperatur oberhalb von 210°C hydriert wird. Zwar werden hierbei Selektivitäten bis zu 100% gefunden, jedoch liegen die Umsätze nur bei maximal 77,5%.

In der Publikation US T892018 ("Defensive Publication") wird ein Verfahren beschrieben, bei dem Isophoron zusammen mit Wasserstoff in eine Reaktionskolonne, die u. a. einen Hydrierkatalysator enthält, geleitet wird. Ein Gemisch aus TMC-on und β-Isophoron wird als Kopfprodukt abgetrennt. Das β-Isophoron in diesem Gemisch wird bei höherer Temperatur isomerisiert. Anschließend wird Rein-TMC-on als Leichtsieder abgetrennt. Das verbleibende α-Isophoron und das Sumpfprodukt der Reaktionskolonne werden wieder in den Prozeß zurückgeführt. Dieses Verfahren hat den Nachteil, daß es insgesamt vier Verfahrensschritte umfaßt.

Da für viele Anwendungszwecke ein qualitativ hochwertiges TMC-on (TMC-on-Gehalt > 99 %; Isophorongehalt (α + β) < 0,1 %) gefordert wird und die bekannten Verfahren hinsichtlich ihrer Wirtschaftlichkeit nicht überzeugen, lag der vorliegenden Erfindung die Aufgabe zugrunde, ein Verfahren bereitzustellen, das es ermöglicht, in wirtschaftlicher Weise hochwertiges TMC-on herzustellen.

Bei der Hydrierung von Isophoron stehen im wesentlichen folgende Verbindungen in thermodynamischem Gleichgewicht:

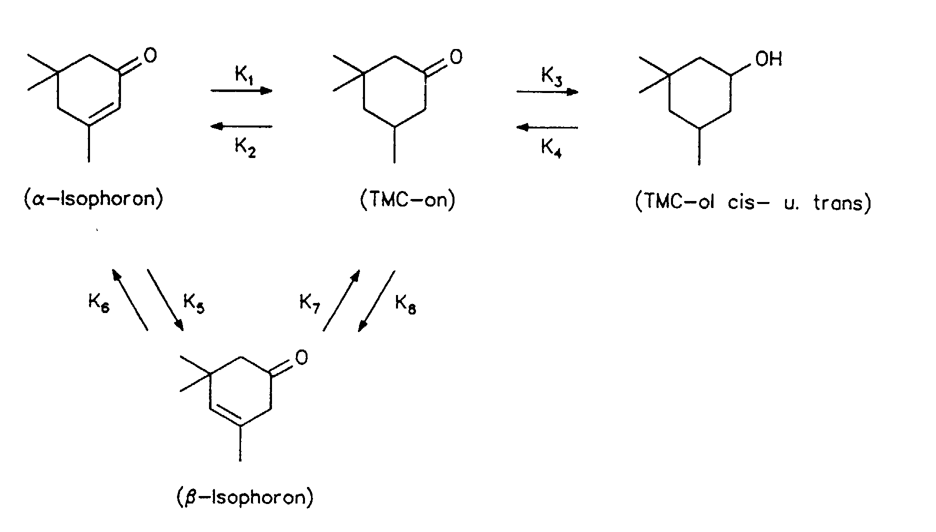

Die Lage des Gleichgewichts hängt u. a. von der Temperatur und dem Wasserstoffdruck ab. Nahezu reines TMC-on dürfte nur unter Bedingungen erhältlich sein, unter denen die Reaktion K₃ praktisch nicht abläuft.

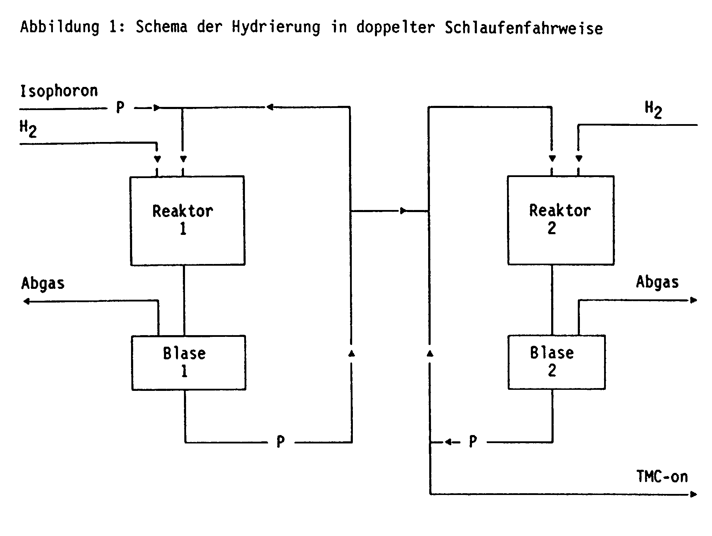

Es wurde nun überraschend gefunden, daß dieses Ziel erreicht wird, wenn die Hydrierung in mehreren hintereinander geschalteten Schlaufen, bevorzugt in doppelter Schlaufenfahrweise (vgl. Abbildung 1), durchgeführt wird. Geeigneterweise wird dabei Isophoron zusammen mit Hydrieraustrag des ersten Reaktors auf den Kopf des ersten Reaktors geleitet. Die dem Frisch-Isophoron äquivalente Menge an Hydriergut des ersten Reaktors wird zusammen mit dem Hydrieraustrag des zweiten Reaktors auf den Kopf des zweiten Reaktors gegeben. Es können auch weitere Schlaufen der zweiten nachgeschaltet werden. Aus der Produktvorlage des zweiten bzw. letzten Reaktors wird standgeregelt Hydriergut abgefahren.

Das vorliegende Verfahren hat den Vorteil, daß technisches Isophoron (α + β-Isophorongehalt ≥ 99,7 Gew.-%), das bis zu 1,6 Gew.-% β-Isophoron enthält, zu einem wasserklaren TMC-on (APHA < 5) mit einem Gehalt > 99 Gew.-% hydriert wird, wobei der Isophoronrestgehalt (Σ α + β) unter 0,1 Gew.-% liegt. Dies entspricht einem Isophoronumsatz von über 99,9 % und einer Selektivität von über 99,3 %.

Besonders vorteilhaft ist, daß sich aufgrund der Qualität des Hydrieraustrags eine Aufarbeitung erübrigt.

Gegenstand der vorliegen Erfindung ist daher ein Verfahren zur Herstellung von 3.3.5-Trimethylcyclohexanon durch katalytische Hydrierung von Isophoron, das dadurch gekennzeichnet ist, daß die Hydrierung in mehreren hintereinander geschalteten Schlaufen durchgeführt wird, wobei Isophoron zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und sofern mehr als zwei Schlaufen vorgesehen werden, der restliche Teil des Hydrierproduktes des 2. Reaktors zusammen mit einem Teil des Hydrierproduktes des folgenden Reaktors auf den Kopf des folgenden Reaktors geleitet wird usw. und das 3.3.5-Trimethylcyclohexanon aus dem restlichen Teil des Hydrierproduktes des letzten Reaktors gewonnen wird, wobei in allen Reaktoren in flüssiger Phase bei einer Temperatur von 30 bis 200°C und bei Drücken von 1 bis 100 bar abs. hydriert wird.

Bevorzugt wird beim erfindungsgemäßen Verfahren die Hydrierung in zwei hintereinander geschalteten Schlaufen durchgeführt, wobei Isophoron zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des zweiten Reaktors geleitet und das 3.3.5-Trimethylcyclohexanon aus dem restlichen Teil des Hydrierproduktes des 2. Reaktors gewonnen wird.

Die Hydrierung kann beim erfindungsgemäßen Verfahren in allen Reaktoren sowohl in turbulenter als auch in laminarer Strömung durchgeführt werden. Es kann auch vorteilhaft sein, die Hydrierung zumindest im ersten Reaktor in turbulenter Strömung durchzuführen. Darüber hinaus wird hier in allen Reaktoren bevorzugt in flüssiger Phase hydriert.

Vorzugsweise führt man die Hydrierung beim erfindungsgemäßen Verfahren bei Temperaturen von 30 bis 200 °C durch, besonders vorzugsweise bei Temperaturen von 40 bis 120 °C. Darüber hinaus kann es von Vorteil sein, die Hydrierung in den Reaktoren auf unterschiedlichen Temperaturniveaus durchzuführen, beispielsweise indem man den zweiten bzw. folgenden Reaktor bei einer niedrigeren Temperatur betreibt als den ersten bzw. vorangehenden Reaktor.

Im allgemeinen betreibt man das erfindungsgemäße Verfahren unter Druck. Bevorzugt wird hier die Hydrierung in den Reaktoren bei Drücken von 1 bis 100 bar abs. durchgeführt, besonders vorzugsweise bei Drücken von 1 bis 20 bar abs. Ferner kann es von Vorteil sein, die Hydrierung in den Reaktoren auf unterschiedlichen Druckniveaus durchzuführen, beispielsweise indem man den zweiten bzw. folgenden Reaktor bei einem niedrigeren Druck betreibt als den ersten bzw. vorangehenden Reaktor.

Als Katalysatorbestückung in den Reaktoren können gängige, hierfür geeignete Hydrierkatalysatoren eingesetzt werden, beispielsweise 0,5 % Pd/Al₂0₃ (Fa. Engelhard).

Bevorzugt wird beim erfindungsgemäßen Verfahren die Hydrierung in den Reaktoren an einem Palladium-Katalysator durchgeführt, besonders vorzugsweise ist dabei der Palladium-Katalysator auf einen Träger aus Aluminiumoxid aufgebracht.

Das erfindungsgemäße Verfahren ermöglicht eine besonders wirtschaftliche Herstellung von hochwertigem TMC-on in exzellenter Ausbeute und hervorragender Selektivität, so daß auch ohne eine zusätzliche Produktaufarbeitung die geforderte Produktqualität erreicht wird.

Das folgende Beispiel soll die vorliegende Erfindung näher erläutern, ohne ihren Anwendungsumfang einzuengen:

### Beispiel 1:

Die Hydrierapparatur (vgl. Abbildung 1) hat zwei baugleiche Reaktoren mit folgenden Abmessungen: 0̸_{c} = 42 mm; 1 = 1 500 mm.
Beide Reaktoren sind jeweils mit 1,9 1 ENGELHARD-Kontakt 0,5 % Pd/Al₂O₃ gefüllt.

Technisches Isophoron (Σ α + β 99,7 Gew.-%) wird verdünnt mit teilhydriertem Isophoron (aus Blase 1) auf den Kopf des ersten Reaktors gefahren (erste Schlaufe).
Die dem eingesetzten Isophoron äquivalente Menge an Hydriergut wird aus Blase 1 standgeregelt in die Schlaufen des zweiten Reaktors gefahren. Aus der Blase 2 wird standgeregelt TMC-on abgefahren.

In den Reaktoren liegen folgende Reaktionsbedingungen vor:

| | Reaktor 1 | Reaktor 2 |
|---|---|---|
| Durchsatz | 1,45 kg/h | ca. 1,46 kg/h |
| Umlauf | 100 kg/h | 80 kg/h |
| H₂-Druck | 15 bar | 10 bar |
| mittlere Temperatur | 100 °C | 40 °C |
| Abgasmenge | 120 Nl/h | 1 Nl/h |

Nach Einstellung des quasistationären Zustandes hat das erhaltene Hydriergut einen TMC-on-Gehalt von 99,1 Gew.-%. Der Restgehalt von Isophoron (Σ α + β) liegt bei 0,05 Gew.-%.

### Vergleichsbeispiel

Im Gegensatz zu Beispiel 1 ist hier der Umlauf des zweiten Reaktors (Umlaufmenge 0) außer Betrieb. Alle anderen Reaktionsbedingungen sind die gleichen wie im Beispiel 1.

Es wird ein Hydriergut mit einem TMC-on-Gehalt von nur 97,6 Gew.-% erhalten. Der Restisophoron-Gehalt (Σ α + β) liegt bei ≤ 0,05 Gew.-%.

## Patentansprüche

1. Verfahren zur Herstellung von 3.3.5-Trimethylcyclohexanon durch katalytische Hydrierung von Isophoron,
dadurch gekennzeichnet,
daß die Hydrierung in mehreren hintereinander geschalteten Schlaufen durchgeführt wird, wobei Isophoron zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und, sofern mehr als zwei Schlaufen vorgesehen werden, der restliche Teil des Hydrierproduktes des 2. Reaktors zusammen mit einem Teil des Hydrierproduktes des folgenden Reaktors auf den Kopf des folgenden Reaktors geleitet wird usw. und das 3.3.5-Trimethylcyclohexanon aus dem restlichen Teil des Hydrierproduktes des letzten Reaktors gewonnen wird, wobei in allen Reaktoren in flüssiger Phase bei einer Temperatur von 30 bis 200 °C und bei Drücken von 1 bis 100 bar abs. hydriert wird.

2. Verfahren nach Anspruch 1,
dadurch gekennzeichnet,
daß die Hydrierung in zwei hintereinander geschalteten Schlaufen durchgeführt wird, wobei Isophoron zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des zweiten Reaktors geleitet und das 3.3.5-Trimethylcyclohexanon aus dem restlichen Teil des Hydrierproduktes des 2. Reaktors gewonnen wird.

3. Verfahren nach Anspruch 1 oder 2,
dadurch gekennzeichnet,
daß die Strömung in allen Reaktoren laminar ist.

4. Verfahren nach Anspruch 1 oder 2,
dadurch gekennzeichnet,
daß die Strömung in allen Reaktoren turbulent ist.

5. Verfahren nach den Ansprüchen 1 bis 4,
dadurch gekennzeichnet,
daß die Hydrierung bei Temperaturen von 40 bis 120 °C durchgeführt wird.

6. Verfahren nach den Ansprüchen 1 bis 5,
dadurch gekennzeichnet,
daß die Hydrierung in den Reaktoren auf unterschiedlichen Temperaturniveaus durchgeführt wird.

7. Verfahren nach den Ansprüchen 1 bis 6,
dadurch gekennzeichnet,
daß die Hydrierung bei Drücken von 1 bis 20 bar abs. durchgeführt wird.

8. Verfahren nach den Ansprüchen 1 bis 7,
dadurch gekennzeichnet,
daß die Hydrierung in den Reaktoren auf unterschiedlichen Druckniveaus durchgeführt wird.

9. Verfahren nach den Ansprüchen 1 bis 8,
dadurch gekennzeichnet,
daß die Hydrierung an einem Palladium-Katalysator durchgeführt wird.

10. Verfahren nach Anspruch 9,
dadurch gekennzeichnet,
daß die Hydrierung an einem Palladium-Katalysator durchgeführt wird, der auf einen Träger aus Aluminiumoxid aufgebracht ist.

## Claims

1. A process for the preparation of 3,3,5-trimethylcyclohexanone by catalytic hydrogenation of isophorone, characterised in that the hydrogenation is carried out in a plurality of series-connected loops, isophorone together with some of the hydrogenation product from the 1st reactor being passed to the top of the 1st reactor, the remainder of the hydrogenation product from the 1st reactor together with some of the hydrogenation product from the 2nd reactor being passed to the top of the 2nd reactor, and, if more than two loops are provided, the remainder of the hydrogenation product from the 2nd reactor together with some of the hydrogenation product from the following reactor being passed to the top of the following reactor, etc., and the 3,3,5-trimethylcyclohexanone being obtained from the remainder of the hydrogenation product from the last reactor, hydrogenation being carried out in all reactors in the liquid phase at a temperature of from 30 to 200°C and at pressures of from 1 to 100 bar absolute.

2. A process according to claim 1, characterised in that the hydrogenation is carried out in two series-connected loops, isophorone together with some of the hydrogenation product from the 1st reactor being passed to the top of the 1st reactor, the remainder of the hydrogenation product from the 1st reactor together with some of the hydrogenation product from the 2nd reactor being passed to the top of the second reactor and the 3,3,5-trimethylcyclohexanone being obtained from the remainder of the hydrogenation product from the 2nd reactor.

3. A process according to claim 1 or 2, characterized in that the flow is laminar in all reactors.

4. A process according to claim 1 or 2, characterized in that the flow is turbulent in all reactors.

5. A process according to any of claims 1 to 4, characterized in that the hydrogenation is carried out at temperatures of from 40 to 120°C.

6. A process according to any of claims 1 to 5, characterized in that the hydrogenation is carried out in the reactors at different temperature levels.

7. A process according to any of claims 1 to 6, characterized in that the hydrogenation is carried out at pressures of from 1 to 20 bar absolute.

8. A process according to any of claims 1 to 7, characterized in that the hydrogenation is carried out in the reactors at different pressure levels.

9. A process according to any of claims 1 to 8, characterized in that the hydrogenation is carried out on a palladium catalyst.

10. A process according to claim 9, characterized in that the hydrogenation is carried out on a palladium catalyst which is applied to a support of aluminium oxide.

## Revendications

1. Procédé de préparation de la 3,3,5-triméthylcyclohexanone par hydrogénation catalytique de l'isophorone,
caractérisé en ce que
l'hydrogénation est effectuée dans plusieurs boucles connectées les unes derrière les autres, l'isophorone étant conduit conjointement avec une partie du produit d'hydrogénation du premier réacteur sur la tête du premier réacteur, dans lequel la partie restante du produit d'hydrogénation du premier réacteur est conduite conjointement à une partie du produit d'hydrogénation du deuxième réacteur sur la tête du deuxième réacteur et pour autant que plus de deux boucles sont prévues, la partie restante du produit d'hydrogénation du deuxième réacteur est conduite conjointement à une partie du produit d'hydrogénation du réacteur suivant, sur la tète du réacteur suivant, etc... et la triméthylcyclohexanone provenant de la partie restante du produit d'hydrogénation du dernier réacteur est produite, procédé dans lequel dans tous les réacteurs on effectue l'hydrogénation en phase liquide à une température allant de 30 à 200°C, et à des pressions allant de 1 à 100 bars abs.

2. Procédé selon la revendication 1,
caractérisé en ce que
l'hydrogénation est effectuée dans deux boucles connectées l'une derrière l'autre, au cours de laquelle l'isophorone est conduite conjointement à une partie du produit d'hydrogénation du premier réacteur sur la tête du premier réacteur, la partie restante du produit d'hydrogénation du premier réacteur est conduite conjointement à une partie du produit d'hydrogénation du deuxième réacteur sur la tête du deuxième réacteur et la 3,3,5-triméthylcyclohexanone provenant de la partie restante du produit d'hydrogénation du deuxième réacteur, est produite.

3. Procédé selon la revendication 1 ou la revendication 2,
caractérisé en ce que
l'écoulement dans tous les réacteurs est laminaire.

4. Procédé selon la revendication 1 ou la revendication 2,
caractérisé en ce que
l'écoulement dans tous les réacteurs est turbulent.

5. Procédé selon les revendications 1 à 4,
caractérisé en ce qui
on effectue l'hydrogénation à des températures allant de 40 à 120°C.

6. Procédé selon les revendications 1 à 5,
caractérisé en ce qu'
on effectue l'hydrogénation dans des réacteurs à des niveaux de température différents.

7. Procédé selon les revendications 1 à 6,
caractérisé en ce qu'
on effectue l'hydrogénation à des pressions allant de 1 à 20 bars abs.

8. Procédé selon les revendications 1 à 7,
caractérisé en ce qu'
on effectue l'hydrogénation dans les réacteurs à des niveaux de pression différents.

9. Procédé selon les revendications 1 à 8,
caractérisé en ce qu'
on effectue l'hydrogénation sur un catalyseur au palladium.

10. Procédé selon la revendications 9,
caractérisé en ce qu'
on effectue l'hydrogénation sur un catalyseur au palliadium qui est appliqué sur un support à base d'oxyde d'aluminium.
